# EUROPEAN PATENT APPLICATION

(11) **EP 2 657 331 A1**
(43) Date of publication of application: **30.10.2013**
(21) Application number: 10861209.4
(22) Date of filing: 22.12.2010
(51) Int. Cl.: C12M 3/00

(54) **CULTURE SUBSTRATE AND CULTURE SHEET**

(71) Applicant: Hitachi, Ltd., Tokyo 100-8280 (JP)
(72) Inventor: TAKAHASHI Ryosuke, Tokyo 100-8220 (JP); HISADA Akiko, Tokyo 100-8220 (JP); SONODA Hiroshi, Tokyo 100-8220 (JP); SAITO Taku, Tokyo 100-8220 (JP); ITABASHI Naoshi, Tokyo 100-8220 (JP); YAMAMOTO Jiro, Tokyo 100-8220 (JP)
(74) Representative: MERH-IP Matias Erny Reichl Hoffmann
(86) International application number: PCT/JP2010/073127
(87) International publication number: WO 2012/086028

(57) **Abstract**

Provided is a culture sheet which enables a technique for forming a three-dimensional tissue having uniform diameter without applying any chemical on the surface of a culture substrate. On the culture sheet (150) of the culture substrate, a plurality of holes (152) are formed and nanopillars (153), which are capable of controlling the adhesiveness and migration ability of cells, are formed on the bottom surface of each hole (152), said bottom face serving as a culture surface. The culture surface of each hole (151) is provided with a partition wall (152) and the internal nanopillars (153) are formed in the vicinity of the center of the hole (151). Owing to this configuration, the interaction among the disseminated cells can be restricted so that uniformly sized three-dimensional structures of the cells can be formed.

## Description

### Technical Field

The present invention relates to a technique of culturing animal and plant cells using a culture substrate, and graphically forming spheroids (3D tissues), and monolayer tissues (2D tissues) of the cells.

### Background Art

In the process of developing pharmaceuticals, in vitro assays using cells instead of animal experiments have been required. In particular, the demand for applying such in vitro assays to the screening and toxicity and metabolism tests of candidate pharmaceutical substances has been increasing.

In such a background, approaches of alternative methods using cells in place of conventional animal experiments has been actively attempted, but many of such approaches have limitations in predicting their clinical reactions. This assumedly because the forms of the cells are not mimicking their actual in vivo structures in these culture methods (Non-Patent Literature 1). Therefore, the construction of 3D tissues which exhibits functions more similar to those of living bodies has been attempted so far, and 3D tissues has been successfully formed for various cell strains.

As a substrate for forming 3D tissues of cells, a sheet (nanopillar sheet) for culture in which regularly arranged ultrafine pillar structures or protrusions are formed on the surface of a sheet has been developed, the 3D tissues formed has the problems that they have high release properties from the substrate (Patent Literature 1), and that they are lost in the process of medium change. Moreover, since it is impossible to control the diameter of formed 3D tissues, it entails the problem that their sizes are not uniform, and therefore the performance of each of the 3D tissues is varied. It is thus still premature as a practical formation method.

To this end, a technique of providing minute cavity structures in a culture substrate, and forming a single 3D tissue per cavity (cellular tissue microchip) has been developed so far (Patent Literature 2, Non-Patent Literature 2). A feature of this technique is that by applying a substance having adhesion to a predetermined region around the center of at the bottom of the cavity, a cell adhesive region and a cell non-adhesive region are defined, and the cavity itself is rotated by a rotation drive apparatus or the like to perform rotation culture, so that cultured cells are retained around the center of the bottom of the cavity which is the cell adhesive region.

### Citation List

### Patent Literature

Patent Literature 1: Japanese Unexamined Patent Application Publication No. 2005-312343
Patent Literature 2: Japanese Unexamined Patent Application Publication No. 2006-121991

### Non-Patent Literature

Non-Patent Literature 1: "The Use of 3-D Cultures for High-Throughput Screening: The Multicellular Spheroid Model"Leoni A. Kunz-Schughart, James P. Freyer, Ferdinand Hofstaedter, and Reinhard Ebner J Biomol Screen, 9: 273-285 (2004)
Non-Patent Literature 2: "Orderly arrangement of hepatocyte spheroids on a microfabricated chip." J Fukuda and K Nakazawa Tissue Eng, 11:1254-62 (2005)
Non-Patent Literature 3: "Formation of Hepatocyte Spheroids with Structural Polarity and Functional Bile Canaliculi Using Nanopillar Sheets." R Takahashi, H Sonoda, Y Tabata and A Hisada, Tissue Eng Part A, 1-45 (Mar 4, 2010)

### Summary of Invention

### Technical Problem

While cellular tissue microchips have such features, in order to compulsorily adhere cells onto specific portions on the surface of the substrate, the cell adhesive region and cell non-adhesive region need to be defined by applying a chemically synthesized substance on the surface of the substrate, which entails some problems.

First, these chemicals applied may adversely affect the growth of cells, but also this operation requires application or adhesion of chemicals in the hyperfine region, which greatly complicates the operation and requires production costs.

Moreover, when inoculated cells fall into non-adhesive regions, they are inevitably disposed of along with the medium when the medium is changed during culture, which is hardly considered as an efficient culture method. Furthermore, it is suspected that the cells which have fallen into the adhesion region are caused to form tissues compulsorily by rotation culture, and therefore stress is exerted on cells, which leads to a lowered activity.

Meanwhile, known nanopillar sheets also have the problems that it is difficult to control the cell movement on the substrate plane, and that it is impossible to control the dimension and diameter of the 3D tissues formed. At the same time, it also has the problem that it is impossible to retain the formed 3D tissues in a target position.

An object of the present invention is to provide a culture sheet, a culture substrate, and a cell culture method using the same which enable forming 3D tissues having a uniform diameter without applying chemicals on the surface of the culture substrate, and further retaining the 3D tissues in a target position.

### Solution to Problem

In order to achieve the above-mentioned object, the present invention provides a culture substrate and a culture sheet in which a culture region is provided, a plurality of projections are formed in the culture region, a partition which partitions the culture region and is taller than the projections around the culture region form, and the constitutional proportion of the projections in the culture region is in the range from 20% to 75%.

Moreover, in order to achieve the above-mentioned object, the present invention provides a culture substrate and a culture sheet in which a culture region is provided, a plurality of projections are formed in the culture region, a partition which partitions the culture region and is taller than the projections around the culture region is formed, and the constitutional proportion of the projections in the culture region is in the range from 40% to 50%.

### Advantageous Effects of Invention

By applying the present invention, formation of 3D tissues can be realized under circumstances with little stress while using only a single material and maintaining their activities by promoting cell movement which is a function inherent to cells.

Moreover, by integrally providing a limited region, i.e., a partition, from the same material, cells inoculated within the limited region are all involved in the formation of a single 3D tissue. This achieves a very efficient culture method, and also leads to the expectation that the sizes of a plurality of 3D tissues formed for the respective limited regions are uniform and homogenous, which is effective in cell assays.

Furthermore, it is expected that the 3D tissues are retained in a target position within the limited region, i.e., the partition. Furthermore, 2D tissues can be formed depending on the purpose. Similar effects are also expected on the 2D tissues.

### Brief Description of Drawings

[Fig. 1] Fig. 1 is a drawing which shows the culture sheet according to Example 1 and the hole structure in the culture sheet.
[Fig. 2] Fig. 2 is an enlarged view which shows the nanopillar structure according to Example 1.
[Fig. 3] Fig. 3 is a drawing which shows a chamber slide according to Example 1, with the culture sheet affixed thereto.
[Fig. 4] Fig. 4 is a drawing which shows the constitution of a plate frame body according to Example 2.
[Fig. 5] Fig. 5 is a drawing for illustrating the flow of the ultrasonic welding of the plate and culture sheet according to Example 2.
[Fig. 6] Fig. 6 is a drawing which shows the flowchart of hepatocyte culture according to Example 3.
[Fig. 7] Fig. 7 is a drawing which shows a photograph of a hepatocyte 3D tissue by the culture sheet by the hepatocyte culture flow according to Example 3.
[Fig. 8] Fig. 8 is a drawing which shows a two-stage and multi-stage nanopillar culture sheet according to Example 4.
[Fig. 9] Fig. 9 is a drawing which shows the types of arrangement patterns of the nanopillars of Examples.
[Fig. 10A] Fig. 10A is a drawing which shows the results of the cell culture (the state of cells) when culture sheets having different pillar diameters shown in Fig. 9 are used.
[Fig. 10B] Fig. 10B is a drawing which shows the results of the cell culture (number of cells formed) when culture sheets having different pillar diameters shown in Fig. 9 are used.
[Fig. 11] Fig. 11 is a drawing which shows an inclined nanopillar culture sheet which is a variant of Example 4 of culture sheets.
[Fig. 12] Fig. 12 is a drawing which shows a well of the culture sheet having a surface tension avoiding pattern, which is variant 4 of Example of the culture sheet.
[Fig. 13A] Fig. 13A is a drawing which shows an appearance perspective view, top view, upper and lower side view of the culture substrate in Example 1.
[Fig. 13B] Fig. 13B is a partially enlarged view of the culture substrate in Example 1, which shows an A-A, B-B partially enlarged view and a C-C, D-D partially enlarged view.
[Fig. 13C] Fig. 13C is a partially enlarged view and end view of the culture substrate in Example 1, and is a drawing which shows an E-E, F-F partially enlarged view, line G-G end view.
[Fig. 14A] Fig. 14A is a drawing which shows a perspective view and bottom view of the appearance of the culture substrate in Example 2.
[Fig. 14B] Fig. 14B is a drawing which shows a top view, upper and lower side view of the culture substrate in Example 2.
[Fig. 14C] Fig. 14C is a partially enlarged view and partial cross-sectional view of the culture substrate in Example 2, which shows an A-A, B-B partially enlarged view, a C-C, D-D partially enlarged view, and an H-H cross-sectional view.
[Fig. 14D] Fig. 14D is a partially enlarged view, and an end view of the culture substrate in Example 2, which shows an E-E, F-F partially enlarged view, and a line G-G end view.
[Fig. 15A] Fig. 15A is a drawing which shows the culture sheet and the hole structure in the culture sheet according to Examples 5 and 6.
[Fig. 15B] Fig. 15B is a schematic diagram which shows an assembly of projection portions having different diameters according to Examples 5 and 6.
[Fig. 15C] Fig. 15C is a drawing which shows an SEM image of the culture sheet of the assembly of projection portions having a diameter of 80 µm according to Examples 5 and 6.
[Fig. 15D] Fig. 15D is a drawing which shows an SEM image of the culture sheet and the assembly of projection portions having a diameter of 20 µm according to Examples 5 and 6.
[Fig. 16A] Fig. 16A is a drawing which shows an example of the distance between the center of a hepatocyte 3D tissue and the center of the hole structure by the culture sheet by the flow of hepatocyte culture according to Example 7.
[Fig. 16B] Fig. 16B is a drawing which shows another example of the distance between the center of the hepatocyte 3D tissue and the center of the hole structure by the culture sheet by the flow of hepatocyte culture according to Example 7.
[Fig. 16C] Fig. 16C is a drawing which shows another example of the distance between the center of the hepatocyte 3D tissue and the center of the hole structure by the culture sheet by the flow of hepatocyte culture according to Example 7.
[Fig. 16D] Fig. 16D is a drawing which shows another example of the distance between the center of the hepatocyte 3D tissue and the center of the hole structure by the culture sheet by the flow of hepatocyte culture according to Example 7.
[Fig. 17] Fig. 17 is a drawing which shows an example of a photograph of the hepatocyte 3D tissue by the culture sheet by the flow of hepatocyte culture according to Example 7.
[Fig. 18] Fig. 18 is a drawing which shows an example of a photograph of the hepatocyte 3D tissue by the culture sheet by the flow of hepatocyte culture.

### Description of Embodiments

The best mode for realizing a method for culturing cells using a culture sheet, and forming the 3D tissues which is a cell cluster, or 2D tissues will be described below in detail.

### Example 1

Example 1 shows an example in which the culture sheet is applied to the chamber slide which is a culture sheet retaining member. Hereinafter, a sheet which has a partition structure which forms the culture region in the present invention on a known nanopillar sheet, and on which a plurality of projections are formed inside the partition structure is referred to as a culture sheet.
The culture sheet is formed from a material which has no adverse effect on cells, in this example, it is polystyrene. However, it goes without saying that the material is not limited to polystyrene.

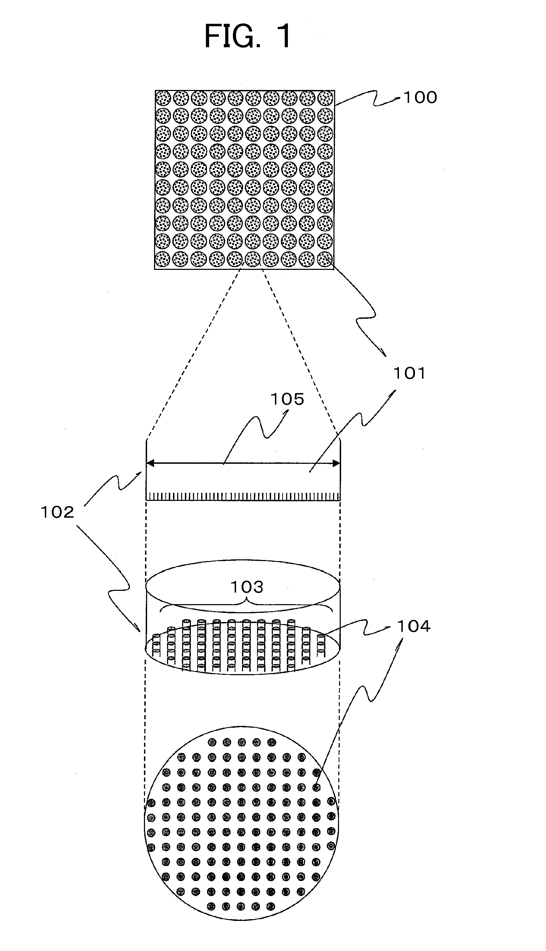

Fig. 1 is a schematic diagram of a scanning electron micrograph of a culture sheet 100 prepared in this Example. Simultaneously, it shows the structure of one of holes 101 (hereinafter referred to as hole) constituted by a plurality of partition structures 102 existing in a single culture sheet. The inside of the hole 101 constitutes a culture region by cell tissue formation unit.

A plurality of projections 102 retained at the bottom of the hole 101 includes a plurality of microprojections 103 (hereinafter also referred to as projections, pillars or nanopillars). Moreover, the diameter of this hole 101 is a hole diameter 105. In the culture sheet 100, the hole 101 including the above-mentioned partition wall 102 and a plurality of projections 103 formed inside the hole 101 are formed from the same material integrally. It should be noted that the shape of this hole 101 is not limited to round, but may have other shapes such as a square shape.

In this manner, the hole 101 and the plurality of projections 103 formed inside the hole 101 including the partition wall 102 are formed integrally as the culture sheet 100 from a single material which has no adverse effects on cells, whereby cells can be grown without foreign substances bonding to the cells in the culture steps. Furthermore, since cells are grown in each of the partitions, cells of a homogeneous size can be formed.

Moreover, a plurality of projections are provided within the partition wall 102 arranged in a surrounding manner, and therefore the cell movement which is the ability inherent to the cells is promoted, and cells are grown by the movement so that cell culture which can maintain the cell activity is possible with no influence of disturbance (stress) by rotation culture or the like.

When a culture region is to be formed while these holes 101 and projection assembly 103 are provided separately, they need to be joined by adhesion or welding.

For example, when these are joined by adhesion, adhesive components enter into the culture region, which may adversely affect generated cells. In joining by welding, the inner diameter of the hole 101 is a hyperfine region diameter on the cell formation level, and therefore it is very difficult to perform welding while forming a target cell region and not damaging the partitions and projections. When the partitions and projections have damages and deformations, unwanted stress may be applied on the cells in the process of cell formation, and the movement of the cells themselves may be impaired.

Therefore, the hole bottom 104, partition wall 102 and projections 103 constituting the holes 101 which forms the culture region are preferably formed integrally. By forming integrally in such a manner, it is preferable because culture excluding the influence of unwanted components other than those required for cell culture can be performed.

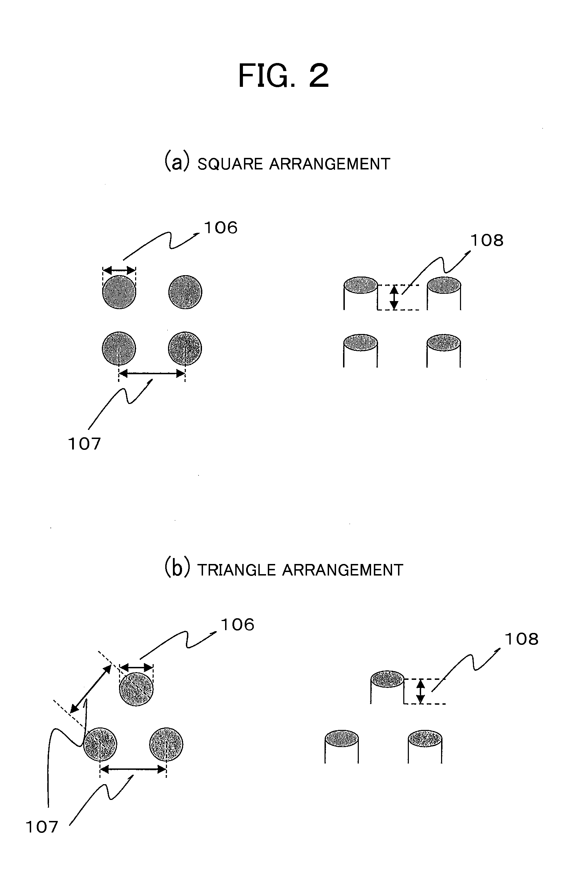

Subsequently, an enlarged view of the projection 103 is shown in Fig. 2. A pillar diameter indicates a diameter 106 of the tip of the projection. A pillar pitch indicates a distance 107 from the center of the tip of the projection to the center of the tip of the adjacent projection. A pillar height indicates a height 108 from the tip of a nanopillar to the bottom thereof. Fig. 2(a) and Fig. 2(b) indicate a square arrangement and a triangle arrangement, respectively, of nanopillars of this Example.

In this Example, culture sheets in which the pillar diameter, pillar pitch and pillar height are 2.0 µm, 4.0 µm, and 1.0 µm, respectively, were used, but as will be described later, such culture sheets are not necessarily used. The height of the partition structure is 70 µm in this Example, but this value is not necessarily used, and suitably the height may be such that the formed cells do not get over the partition.

The culture sheet 100 in this Example is produced by the method described below. A mold in which round holes each having a diameter of 200 µm and depth of 70 µm are arranged in the form of squares, and micropores each having a diameter of 2.0 µm and a depth of 1.0 µm are formed at the bottom at a pitch of 4.0 µm was pressed against a polystyrene film having a thickness of 400 µm at 135°C and a pressure of 2 MPa. The film was took out from a press machine after being cooled to room temperature, and the mold was peeled off from the polystyrene film, whereby a culture sheet retaining a plurality of holes each having a hole diameter of 200 µm and having a plurality of projections at the bottom thereof can be produced.

Herein, a mold material is silicon wafer, and in order to prevent adhesion with the polystyrene film during the production of the culture sheet, a mold releasing process is performed in advance with a fluorine-based mold releasing agent. Silicon wafer was used as the mold material in this Example, but a mold made from other metal materials and the like may be also used.

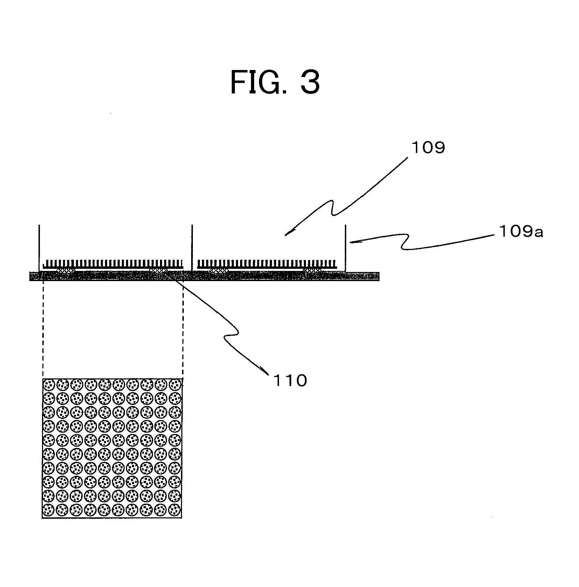

As shown in Fig. 3, the culture sheet 100 produced by integral molding from the single material in this manner was cut into 2-cm square pieces in this example, and a surgical glue 110 was applied onto the glass bottom of the chamber slide 109 to adhere the chamber slide 109 and the culture sheet 100, whereby the chamber slide 109 with the culture sheet 100 affixed thereto is produced. It should be noted that in Fig. 3, 109a represents a frame for partitioning the culture sheets 100. This frame 109a is formed from, for example, a plastic material or the like. It should be noted that the shape of a frame body such as this frame 109a is not limited to square, but may be other shapes such as a round shape.

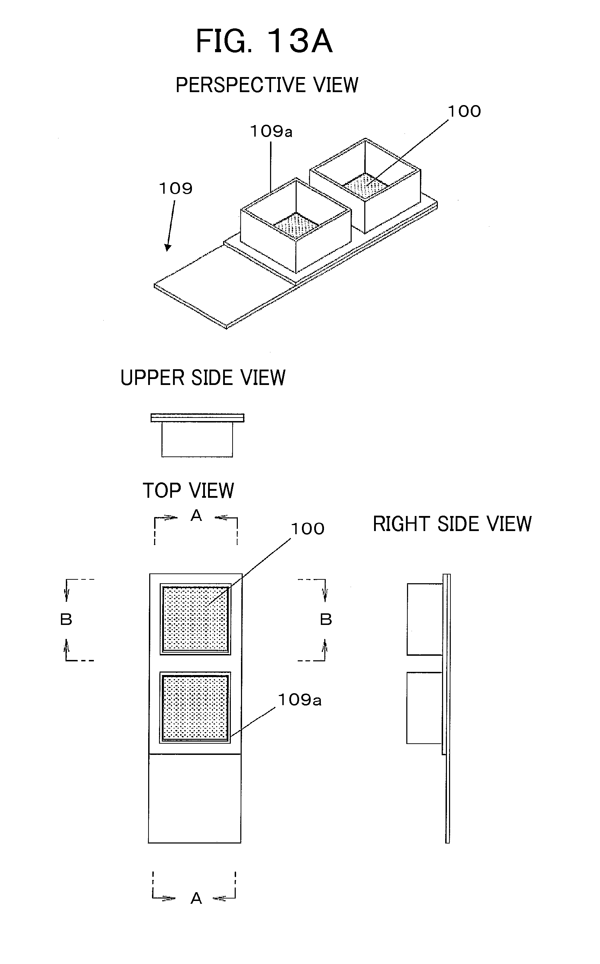

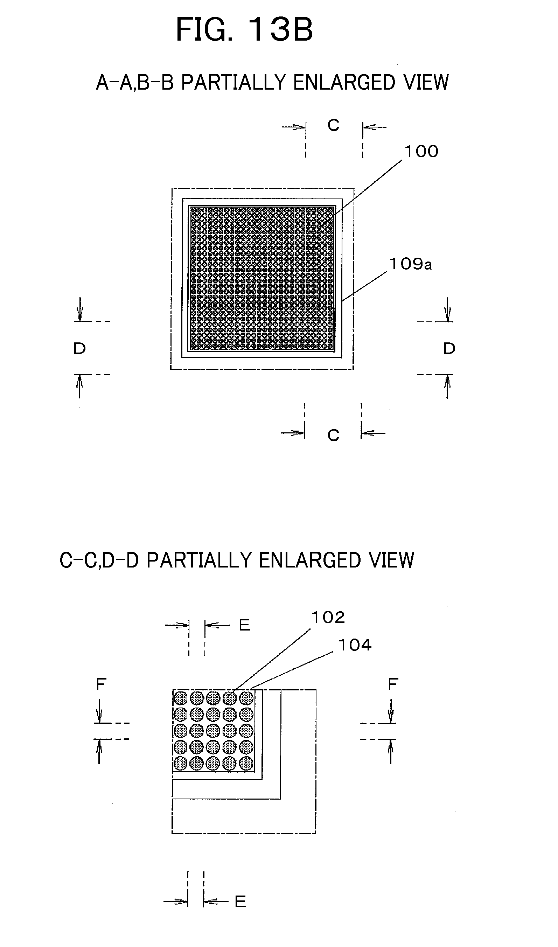

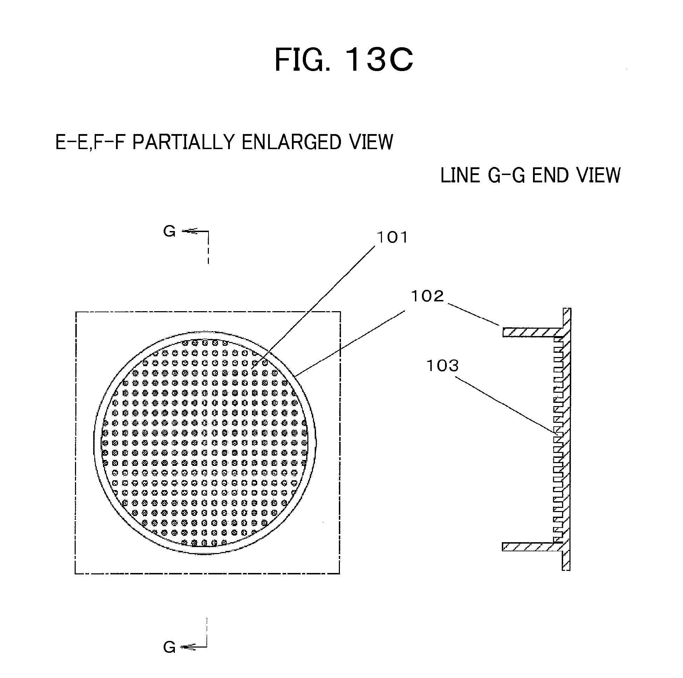

Figs. 13A, 13B, 13C, show the overall constitution diagram and principal part cross-sectional view of the chamber slide with the culture sheet of this Example affixed thereto.
Fig. 13A is an appearance perspective view, top view, and upper and lower side views of the culture substrate in this Example. Illustration of left and right side elevational views is omitted since its form is obvious from the perspective view.
Fig. 13B is a partially enlarged view, which shows an A-A, B-B partially enlarged view, and a C-C, D-D partially enlarged view.
Fig. 13C is a partially enlarged view and end view, which shows an E-E, F-F partially enlarged view, and a line G-G end view.

The article shown in Figs. 13A to 13C is a culture device (culture containers) for culturing cells of humans, animals, plants and others, and are each constituted by the culture sheet 100 and a retaining member (chamber slide) 109 which retains the culture sheet 100. A plurality of partition portions 102 are formed on the surface of the culture sheet 100, and is provided at the bottom of the inside of a cylindrical hole portion 109a formed on the retaining member 109.

Furthermore, culture regions having a plurality of minute projection portions 103 within the partition portion are formed respectively. When target cells to be cultured are added to the inside of the hole portion 109a, as added to the sheet surface forming the culture regions within the partition portion 102, the target cell is retained in the plurality of minute projection portions 103 and cultured.

### Example 2

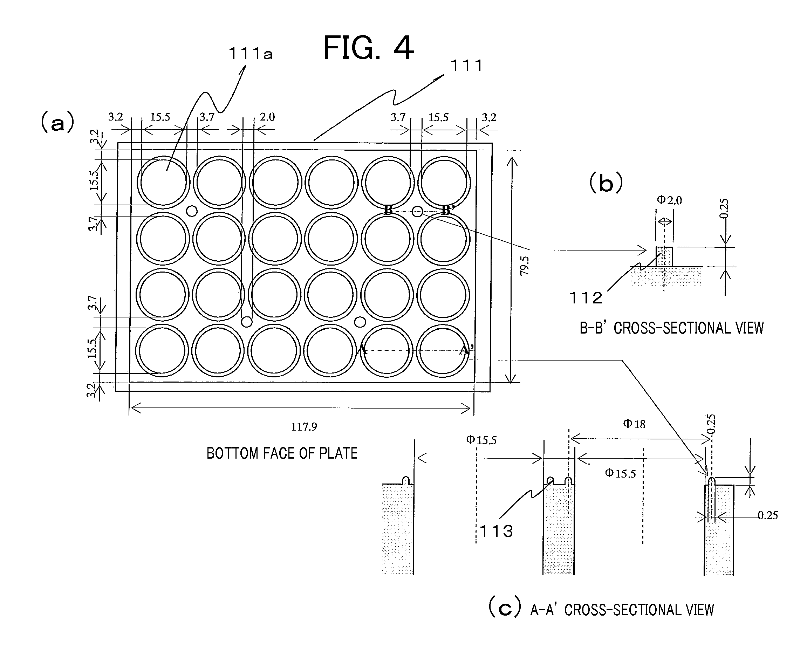

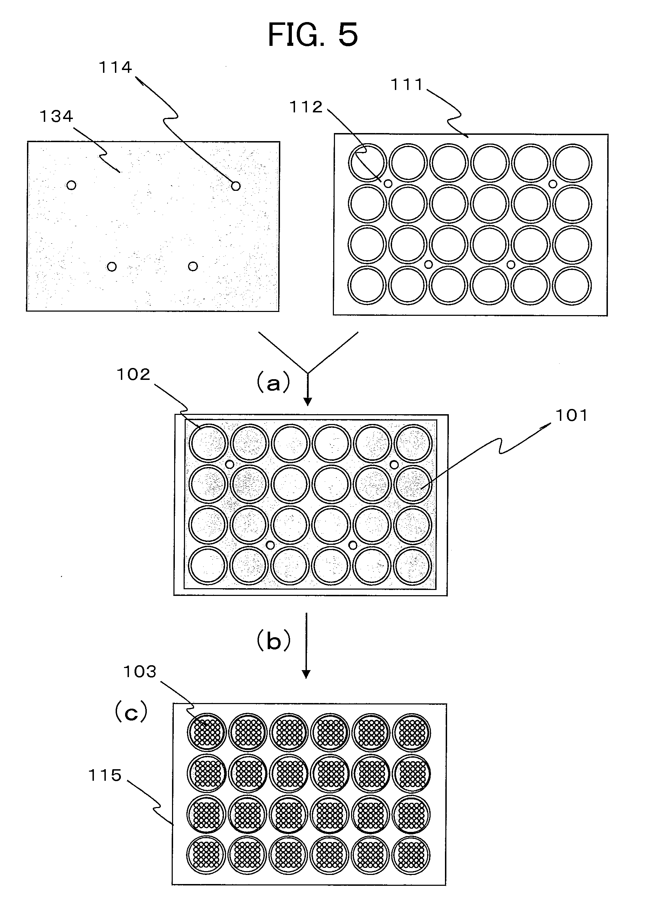

Subsequently, Example 2 will be described with reference to Figs. 4 and 5. In Example 2, the constitution of a multiwell plate with a culture sheet and a production example thereof will be shown. Fig. 4(a) is a bottom view of a frame body 111 constituting the multiwell plate. The frame body 111 which is a culture sheet retaining member is such that has 24 cylindrical hole portions 111a in total, arranged in 4 rows and 6 columns, formed in an area measuring about 125 mm in width, about 80 mm in length, and about 20 mm in height. The material used is polystyrene.

The number of holes formed on the frame body normally ranges from 6 to 1536, varied depending on the use, and therefore the number of holes on this frame body is not limited to 24. The material of the frame body is not limited to polystyrene either.

In producing the culture substrate, the frame body 111 and the culture sheet 100 is joined by ultrasonic welding.

The following processes are performed on the frame body 111 in advance. As the first process, a projection for fixing film 112 is processed at the bottom of the frame body 111 for the purpose of preventing the cell culture sheet and the plate from being shifted due to the vibration of ultrasonic waves provided when the frame body 111 and the culture sheet 100 are welded. As the second process, a rib structure 113 is provided to weld the culture sheet by ultrasonic waves.

Figs. 4(b) and 4(c) are shows the cross-sectional views at lines B-B' and A-A', respectively, in Fig. 4(a). Moreover, holes 114 having the same diameter are provided in the culture sheet in the same position when both are overlapped so that the projection engages with the projection for fixing film. Successively, this frame body and the culture sheet 100 are adhered by ultrasonic welding.

The step of the welding is shown in Fig. 5. First, the holes of the projection for fixing film of the frame body and of the culture sheet are placed together and stacked (Fig. 5(a)). Subsequently, ultrasonic waves are produced from the culture sheet side from an ultrasonic wave oscillator via a converter, a booster, or further a horn, and both are welded (Fig. 5(b)). A horn is an apparatus for welding by irradiating an appropriate position with ultrasonic waves of an appropriate energy. A specific apparatus designed so that ultrasonic waves are generated appropriately along the position of the rib structure was produced and used. 115 shows a top view of the thus-produced plate.

The frame body and the culture sheet were joined by using ultrasonic welding in this Example, but it goes without saying that the joining is not limited to this method. Formation of a plate can be realized without any intervention of organic matters such as adhesives which affects cells by ultrasonic welding. Therefore, no adverse effects are caused on cells. Needless to say, this Example is a culture sheet which is applicable and useful not only to toxicity and metabolism tests in new drug development processes, but also to the formation of organizations intended for regenerative medicine.

It is needless to say that by providing a plurality of the rib structures at the bottom of the frame 109a also in the culture substrate of the chamber slide shape shown as an example in Example 1, and performing welding with the culture sheet 100 by the rib structures, the culture substrate can be produced by a joining method similar to this Example.

In the culture substrate prepared in this manner, a plurality of the holes 101 are formed on the culture sheet 100 formed at the bottom of the frame body 111, and a plurality of projections constituted at the bottom 104 of the hole include a plurality of microprojections 103 (hereinafter also referred to as projections, pillars or nanopillars). Moreover, the diameter of this hole 101 is used as a hole diameter 105. In the culture sheet 100, the hole 101 including the above-mentioned partition wall 102 and the plurality of projections 103 formed inside the hole 101 are formed from the same material integrally. It should be noted that the shape of this hole 101 is not limited to round, but may have other shapes such as a square shape.

In this manner, the hole 101 including the partition wall 102 and the plurality of projections 103 formed inside the hole 101 are formed integrally from a single material which has no adverse effects on cells as a culture sheet, whereby cells can be grown with no foreign substances adhering to cells in the culture step. Furthermore, since cells are grown in each of the partitions, cells of a homogeneous size can be formed.

Moreover, a plurality of projections are provided within the partition arranged in a surrounding manner, and therefore cell movement, which is the ability inherent to the cells, is promoted, and cells are grown by the movement so that cell culture which can maintain the cell activity is possible with no influence of disturbance (stress) by rotation culture or the like.

When a culture region is to be formed while these holes 101 and projection assembly 103 are provided separately, they need to be joined by adhesion or welding. For example, when joined by adhesion, adhesive components enter into the culture region, which may adversely affect generated cells.

Moreover, when welding is to be performed, the inner diameter of the hole 101 is a hyperfine region diameter on the cell formation level, and therefore it is very difficult to perform welding while forming a target cell region and not damaging the partitions and projections. When the partitions and projections have damages and deformations, unwanted stress may be applied on the cells in the process of cell formation, and the movement of the cells themselves may be impaired.

Therefore, the hole 101 which forms the culture region and the projections 103 are preferably formed integrally by forming integrally in such a manner, it is preferable because culture excluding the influence of unwanted components other than those required for cell culture can be performed.

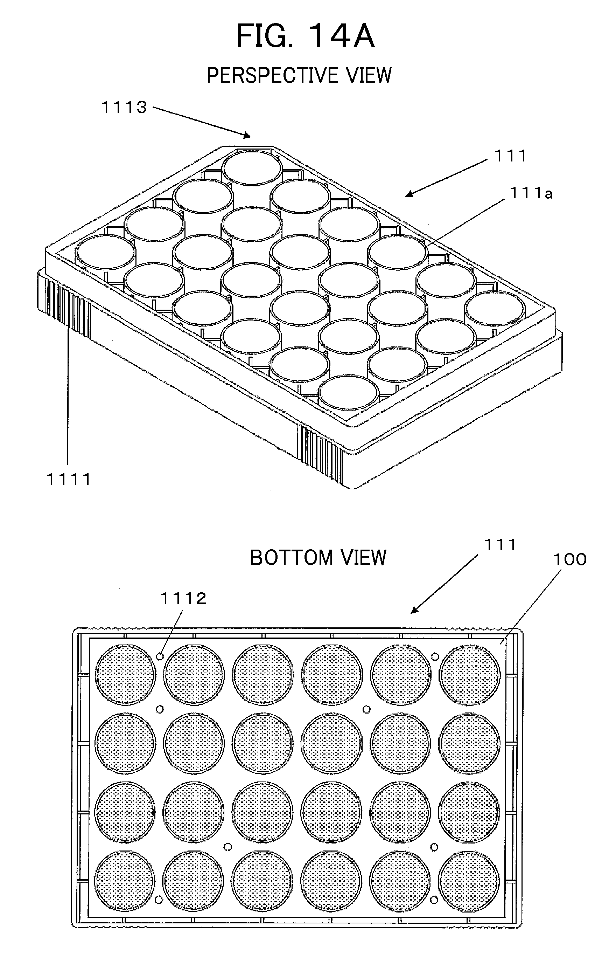

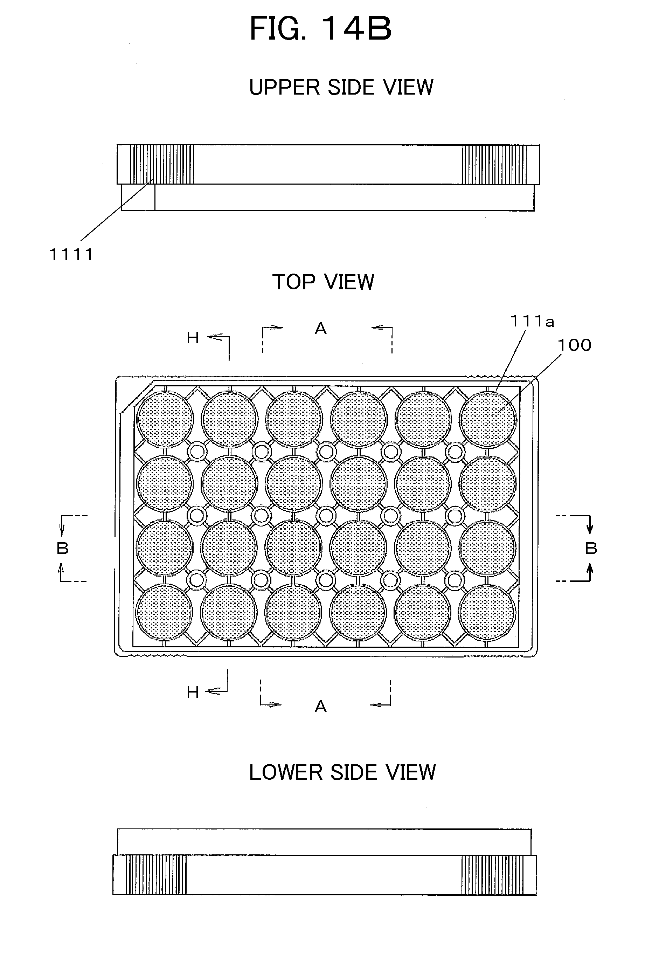

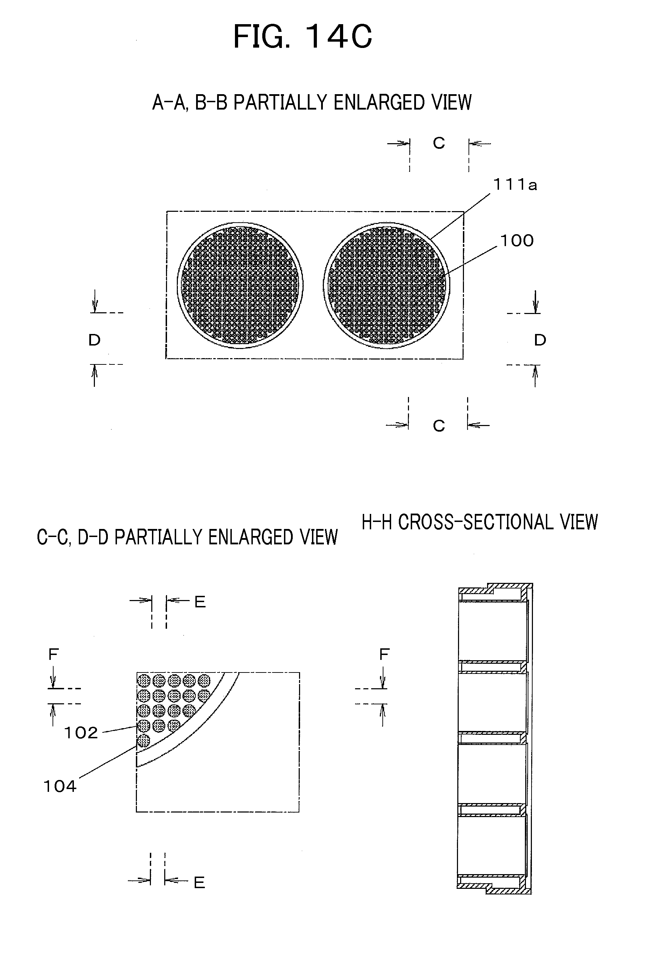

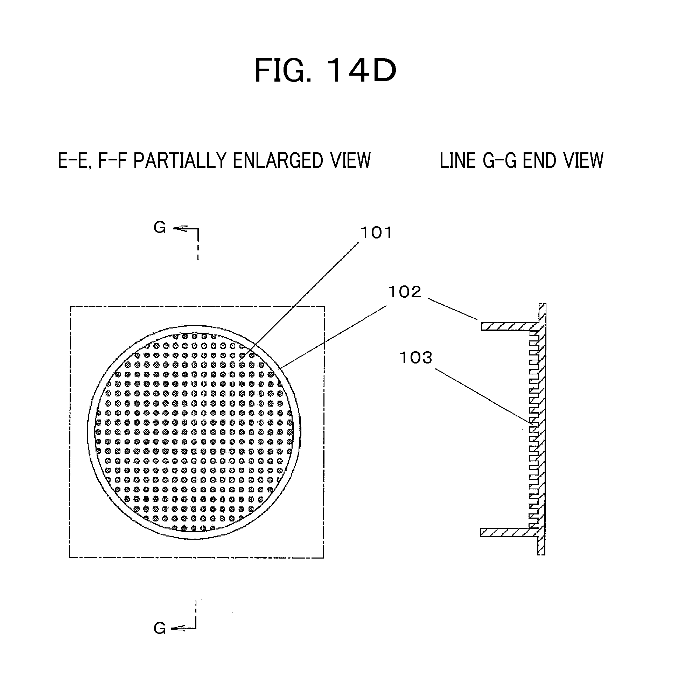

Herein, in Figs. 14A, 14B, 14C, and 14D, an overall constitution diagram and a principal part cross-sectional view of a multiwell plate with the culture sheet of this Example are shown.

Fig. 14A shows an appearance perspective view and a bottom view of the culture substrate in this Example.
Fig. 14B shows a top view and upper and lower side views of the culture substrate. Herein, illustration of left and right side elevational views is omitted since its form is obvious from the appearance perspective view.
Fig. 14C is a partially enlarged view and a partial cross-sectional view, which show an A-A, a B-B partially enlarged view, a C-C, D-D partially enlarged view, and an H-H cross-sectional view.
Fig. 14D is a partially enlarged view, and an end view, which show an E-E, F-F partially enlarged view, and a line G-G end view.

The article shown in Figs. 14A, 14B, 14C, 14D is a culture device (culture container) for culturing cells of humans, animals, plants and others, and is constituted by the culture sheet 100 and a retaining member (frame body) 111 which retains the culture sheet 100.

A plurality of the holes 101 are formed on the surface of the culture sheet 100, and is provided at the bottom of the inside of a cylindrical hole portion 111a formed in the retaining member.
Furthermore, culture regions having a plurality of minute projection portion 103 within the partition portion are formed respectively. When target cells to be cultured are added to the inside of the hole portion 111a, as added to the sheet surface forming the culture regions within the hole 101, the target cell is retained in the plurality of minute projection portions 103 and cultured.

Moreover, the culture substrate of this example shows an example in which the culture sheet is welded from the back side of the frame body 111, and the frame body 111 which is a retaining member and the culture sheet 100 are welded via a joint 1112.
The joint 1112 is provided on the outside of the hole portion 111a, and the culture region is not affected by the welding.
Therefore, although welding was shown as an example in this example, the joining method is not limited to this, and other joining methods can be also employed since joining does not affect the culture region itself with other joining methods.

In addition, in the substrate of this example, the frame body 111 has the form of a square, and at least of the four apexes is cut off. The formation of this cut surface 1113 facilitates specification of the position of the hole portion of the substrate by the operator who performs culture.
This cut face is not essential, and of course may be or may not be present. A non-slip portion 1111 is formed on the culture substrate, which can prevent the operator from unexpectedly shaking and dropping the substrate and prevent other accidents during the operation.

### Example 3

Example 3 shows an example of application of cells to tissue culture using the culture substrates produced in Examples 1 and 2. In the development of new drugs, the construction of the 3D tissues which reflects biological functions has demands for various evaluations utilizing cells substituting animal experiments. In addition, when the thus-formed 3D tissues are subjected to various tests in screening of pharmaceuticals or development of new drugs, it is necessary to verify in advance whether the 3D tissues retain activities to withstand the tests. In this case, if the formed spheroids are held in the predetermined position with high reproducibility, it is expected that they are suitable for high through-put screening and various tests.

Moreover, before culturing induced pluripotent stem cells (iPS cells) and embryonic stem cells (ES cells) to cause them to differentiate into target cells, 3D tissues need to be formed. Therefore, a technique of easily constructing 3D tissues has been demanded also in the field of regenerative medicine. From such a background, an example of forming 3D tissues using the chamber slide in particular is shown herein, but the essential part of cell culture is not especially different even for a multiwell plate. In this example, an example using rat hepatocytes is shown, but as mentioned above, it is applicable to cell strains of various animals and plants, and cell strains are not especially limited.

Preparation of hepatocytes is performed according to in situ collagenase perfusion technique. The detail is as follows: The abdomen of a Fisher 344 male rat (7 to 10 weeks old) is opened under pentobarbital anesthesia, and a catheter is inserted into the portal vein to inject a preperfusate (Hanks' solution not including Ca²⁺ or Mg²⁺ and containing EGTA).

The postcaval vein in the lower liver is simultaneously incised to discharge blood. Next, the thorax is opened, the postcaval vein which goes into the right atrium is incised, and the postcaval vein in the lower liver is clipped with a clamp to perform perfusion. Perfusion is stopped after it is confirmed that the blood removal from the liver has been fully conducted. The perfusate is exchanged to a collagenase solution to perform perfusion.

Perfusion is performed using the Hanks' solution containing 0.05% of collagenase in this example, but this solution is not necessarily used. Perfusion is stopped after it is confirmed that intercellular tissues have been digested by collagenase. The liver is separated, cut into small pieces in a cooled Hanks' solution, and is dispersed into cells by pipetting. Subsequently, undigested tissues are removed by gauze filtration. The cell suspension is repeatedly centrifuged at 50 G for 1 minute several times to remove nonparenchymal cells. Subsequently, damaged hepatocytes are removed by centrifugal separation at 500 G for 5 minutes using an isotonic Percoll solution. The survival rate of the obtained hepatocytes is measured by the trypan blue exclusion method, and the hepatocytes with a survival rate of 85% or higher are used for culture. Herein, the hepatocytes with a survival rate of 85% or higher are used for culture, but it goes without saying that this condition is not necessarily used. Preparation of the hepatocytes is not necessarily limited to the in situ collagenase perfusion technique.

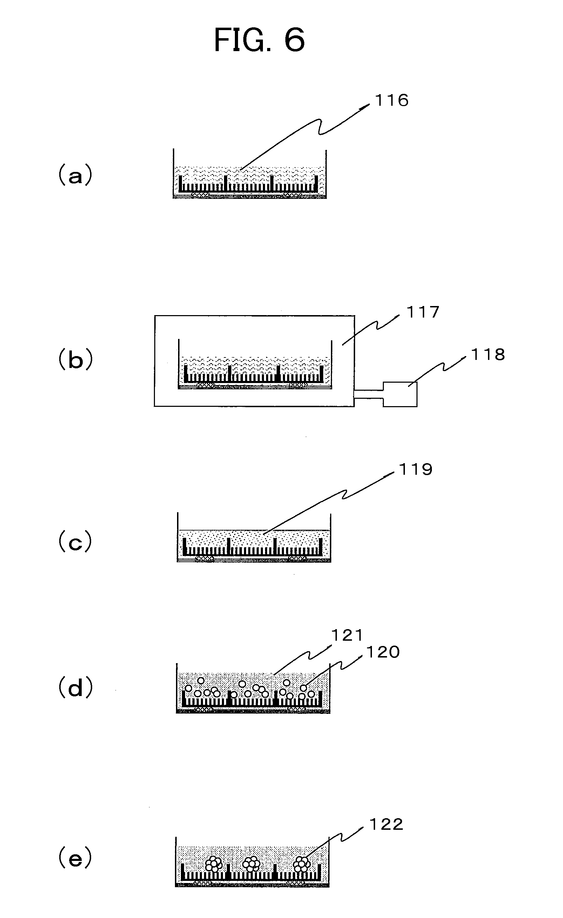

A flowchart of the culture using the thus-obtained hepatocytes is shown in Fig. 6.

In the flowchart of Fig. 6, first, type I collagen 116 is applied to the culture sheet of the chamber slide type produced in Example 1. A 1 to 1.5-ml portion of a diluted solution which has been produced by diluting type I collagen dissolved in a weakly acidic solution with sterile water to a predetermined concentration is added to the chamber slide mentioned above (Fig. 6(a)). Next, a decompression operation is performed in order to cause the added type I collagen to be adsorbed onto the nanopillar sheet 100 completely (Fig. 6(b)). The decompression operation is performed at 0.04 atmosphere or lower using a decompression container 117 and a decompression pump 118. The decompression time is not particularly limited, but the decompression is performed for 10 minutes in this Example. The constitution of the apparatus used for decompression is not particularly limited. Herein, the range of the predetermined concentration of the diluted solution is 100 (ng/ml) or higher and 10 (µg/ml) or lower. The concentration is not necessarily limited to this range, but this range is suitable for spherical 3D tissues to form. Finally, an excess of type I collagen is removed, and PBS(-) 119 is added thereto (Fig. 6(c)). This operation is performed three times, and an excess of type I collagen is washed.

Hepatocytes 120 prepared by the in situ collagenase perfusion technique as above-mentioned are suspended in a medium 121, and the suspension is inoculated on the NP sheet with Type I collagen prepared as stated above applied thereto similarly (Fig. 6(d)). The medium is not particularly limited, but, a Williams E medium including a medium containing serum (FCS), insulin, and dexamethasone (hereinafter referred to as medium (including 10% FCS)) is used. In this Example, a Williams E medium containing 10% FCS, 8.6 nM insulin, and 255 nM dexamethasone is particularly used. After inoculation, culture is started using a CO₂ incubator under the conditions of 5% CO₂ and 37 °C, the first medium exchange is performed after 18 hours or more has elapsed, and medium exchange is performed every 24 hours henceforth. Although the medium used for the culture after the 18th hour after the inoculation is not particularly limited, in this example, a medium (hereinafter referred to as medium (containing no FCS) with FCS removed from a medium (containing 10% FCS) is used.

Moreover, the inoculation density of hepatocytes was set to 1×10⁵ cells/ml in this Example, but is not limited to this concentration. Herein, the culture sheet 100 used for culture has a pillar height, pillar diameter and pillar pitch of 1.0 µm, 2.0 µm, and 4.0 µm, respectively, but the values are not limited to these.

Moreover, the concentration of Type I collagen added to the culture sheet is set to 100 (ng/ml) in this Example, but may be a concentration other than this. Spheroids may be formed at a concentration other than this concentration depending on the conditions of the cells. The cells are cultures for 96 hours in total, whereby 3D tissues 122 are formed (Fig. 6(e)).

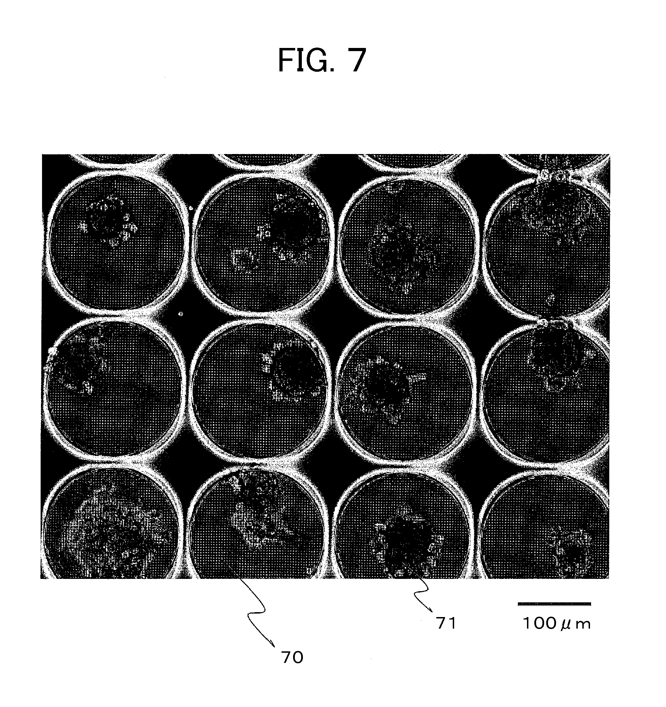

Fig. 7 shows a photograph of the results of actual culture of hepatocytes using the above-mentioned culture sheet having a hole diameter of 200 µm. As can be seen from Fig. 7, spherical 3D tissues 71 having such similar sizes are formed in the holes 70 with no special chemical applied onto the surface of the culture sheet and by stationary culture having little stress on cells. This culture method supposedly does not deteriorate the activity of the cells originally retained, and is therefore effective for cell assays and the like.

### Example 4

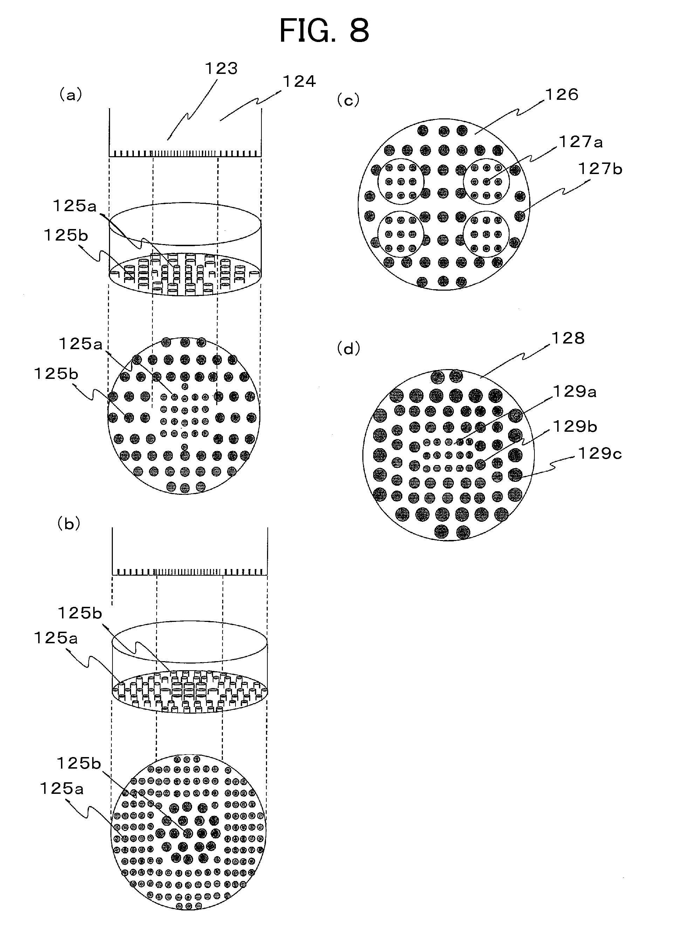

Fig. 8 shows a variant of Example of the culture sheet 100 mentioned above as Example 4. First, an example is shown in which in a culture sheet 123, by arranging the arrangement pattern of projections which provides differences in the migration and adhesion of cells in two stages, as in Fig. 8(a), in a manner of surrounding a first arrangement pattern 125a with a second arrangement pattern 125b, 3D tissues or 2D tissues are formed on the first arrangement pattern 125a (for example, near the center of the hole).

Contrarily, an example is shown in which, as in Fig. 8(b), by arranging in two stages in a manner of surrounding the second arrangement pattern 125b with the first arrangement pattern 125a, 3D tissues or 2D tissues are formed on the second arrangement pattern 125b (for example, the periphery of the hole). It should be noted that 124 represents a hole as in the preceding Examples. The dotted line indicates the boundary of the patterns.

By arranging the first arrangement pattern not only in the central portion of the hole 124, but also arranging the same as in the culture sheet 126 of Fig. 8(c), by surrounding, for example, 4 portions of the first arrangement patterns 127b with the second arrangement pattern 127a, tissues having similar sizes can be formed on the first arrangement pattern. In this manner, the combination of the pillar diameter, pillar pitch, and arrangement pattern can be an optimum pattern of arrangement depending on the purpose to perform culture. Similarly, Fig. 8(d) shows a culture sheet 128 in which the arrangement pattern is set to be multi-stage patterns 129c, 129b, 129a.

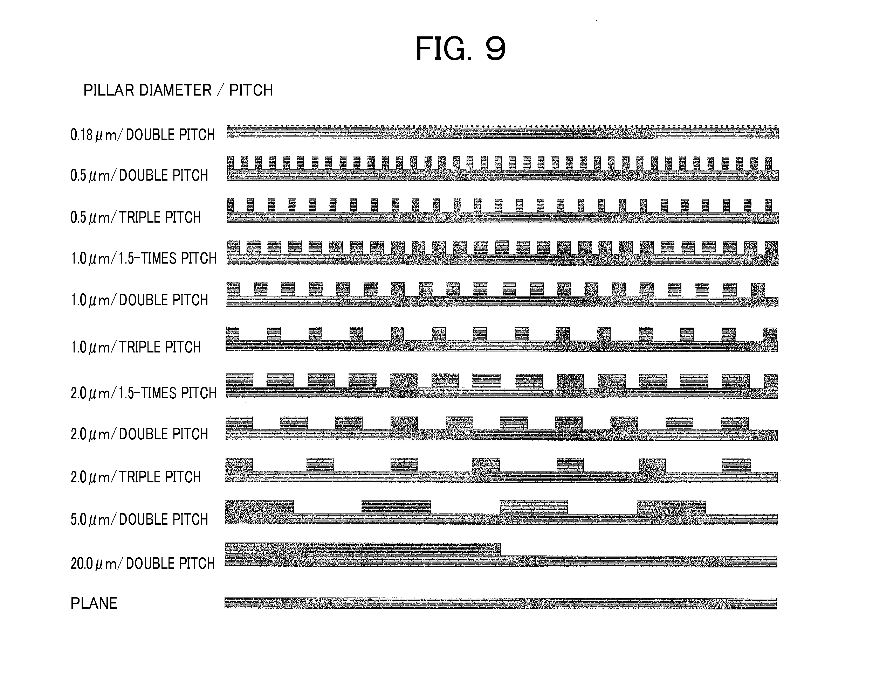

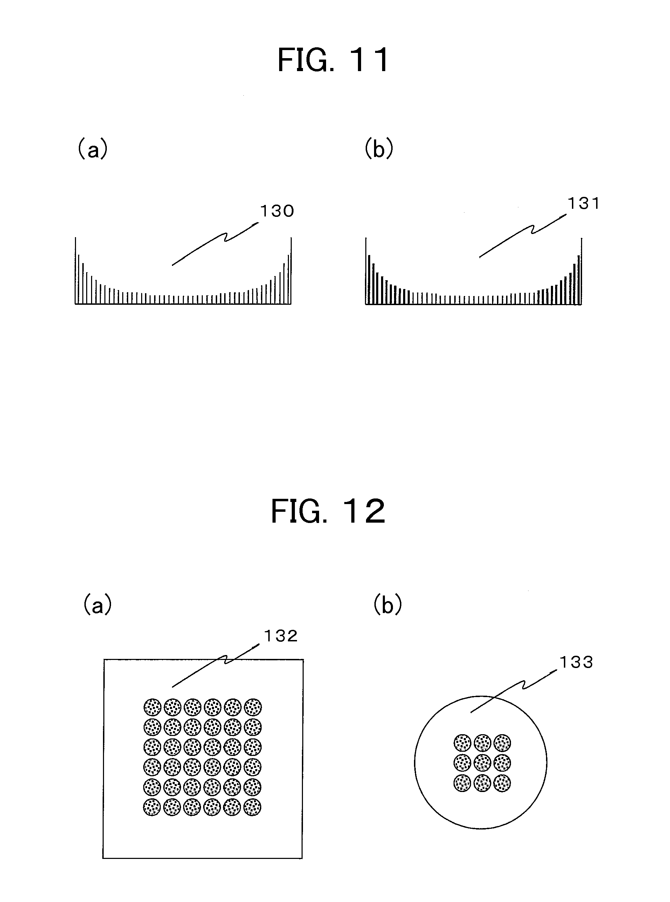

Next, using Fig. 9, the types of the arrangement patterns (hereinafter referred to as pillar patterns) of the projections in the above-mentioned Example will be described. As shown in Fig. 9, 11 types of arrangement patterns have been shown as examples. As can be seen from the same figure, there are 11 types of arrangement patterns with the pillar diameter and pillar pitch ranging from 0.18 to 20.0 µm and from 0.36 to 40.0 µm, respectively, but the pillar diameter and pillar pitch are not limited to these.

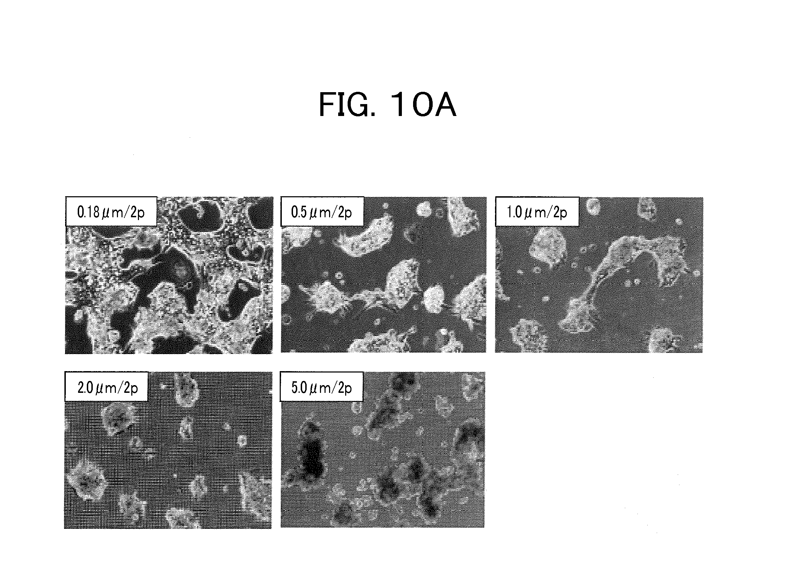

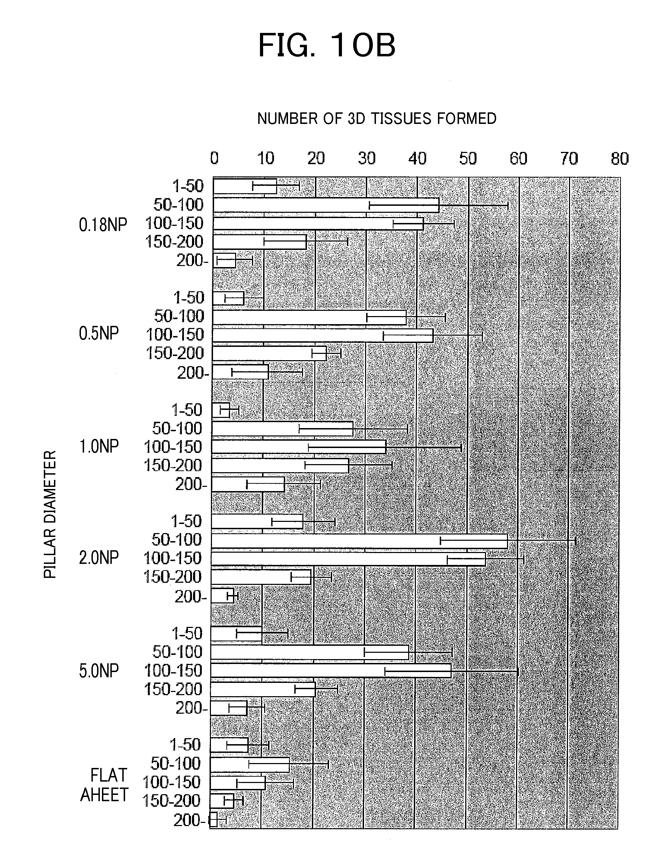

An example of hepatocytes cultured under these pillar patterns is shown in Figs. 10A and 10B.

It should be noted that in the culture on a flat plane with no pillar pattern, many cells are discharged along with the medium when the medium is changed during the culture, and therefore desired cultured cells cannot be effectively obtained. Accordingly, no illustration is provided in Fig. 10A.

Fig. 10A are figures which show the states of the cells when culture is performed using the culture sheet 100 with the double pitch relative to the pillar diameter. As a result, when the pillar diameter is 0.18 µm, 0.5 µm, and 1.0 µm, flat tissues which are not spherical are adhered at the bottom of the substrate, while, when it is 2.0 µm and 5.0 µm, 3D tissues which are spherical are formed on the substrate.

Comparing the spherical cells formed in the substrate with the pillar diameter of 2.0 µm and 5.0 µm, the substrate with the pillar diameter of 2.0 µm had more cells adhered onto the substrate, indicating that it is in a stable state. That is, it can be seen that as for the cell adhesion, the greater the pillar diameter, the lower the adhesion and the more promoted the movement by cells.

Fig. 10B is a graph which shows, as for the number of 3D tissues (spheroids) of the hepatocytes formed on the sheets with each of the pillar diameters, the results grouped by diameter of the spheroids formed. The area of the sheet is 4 square cm (2 cm × 2 cm).

In the 3D tissues of hepatocytes, in cell assays intended for drugs screening, and the toxicity and metabolism tests which can substitute animal experiments in the innovative drug development field, cells having diameters of 50 to 100 microns are preferable. In this example, it can be seen that the number of the formed cells of this size is the most in the case of the substrate with a pillar diameter of 2.0 µm, indicating that this pillar diameter is preferable.

However, under the above-mentioned examination, it was stated that the case where the pillar diameter is 2.0 µm is preferable in order to form cells having diameters of 50 to 100 microns, but the pillar diameter is not limited to this, and for all the pillar diameters used in this examination, it was found that a greater number of cells with stable shapes are formed compared to the flat state with no pillar formed. Thus, the form or adhesion to the substrate of cells or tissues formed from cells can be freely changed by the difference in pillar pattern.

By applying the results stated above, as explained in Example of Fig. 8, by arranging in two stages in a manner of surrounding the first arrangement pattern with a small pillar diameter (pillar pitch) by the second arrangement pattern with a large pillar diameter (pillar pitch), or arranging in multiple stages, tissues having target shapes can be formed in target positions within the holes utilizing cell adhesion and the motion characteristics of cells themselves.

Moreover, by decreasing the heights of the nanopillars having the same size of the pillar diameter from the periphery toward the central portion of the hole, it is possible to provide a difference in height gradually in a manner of inclining, to promote the movement of cells so that they gather in the central portion by gravity and form tissues.

Fig. 11(a) shows a culture sheet 130 which is a variant in which a difference is provided in the heights of the nanopillars gradually. At this time, unlike in a normal U-shaped culture container, there is produced an effect that the cells are retained in the center by the presence of the pillars. In addition, as in the culture sheet 131 of Fig. 11(b), it is also possible to promote the effect stated above by providing a difference in pillar diameter even in the inclination.

In the variant of Fig. 11, the height is changed gradually to smoothen the inclination, but a constitution in which the height is sequentially changed stepwise may be also employed.

Moreover, a plurality of holes gather to form a culture surface (square shape in the case of the chamber slide, round shape in the case of and the plate), but in the culture, a difference occurs in how 3D tissues are formed in the central portion and peripheral portion of the culture surface by the influence of the surface tension. That is, although 3D tissues are formed in the central portion of the culture surface, 3D tissues may not be formed in some events for the reason that the amount of the medium is increased of the portion by the surface tension in the peripheral portion, the amount of oxygen supplied is lowered, or the high water pressure is applied. In order to avoid this phenomenon, the culture sheets 132, 133 retaining the hole structure may be produced only in the central portion of the culture surface as shown in Figs. 12 (a), (b).

By forming the culture sheet in this manner, the culture substrate having high culture efficiency and little production load can be achieved.

### Example 5

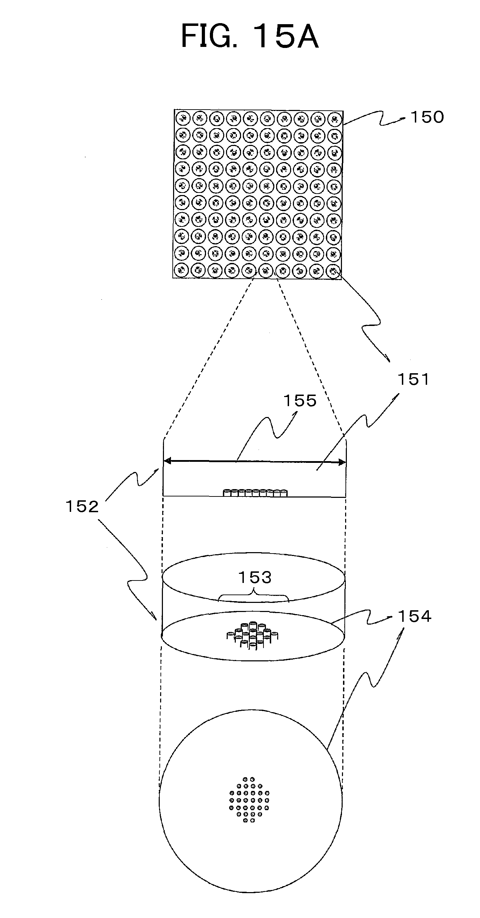

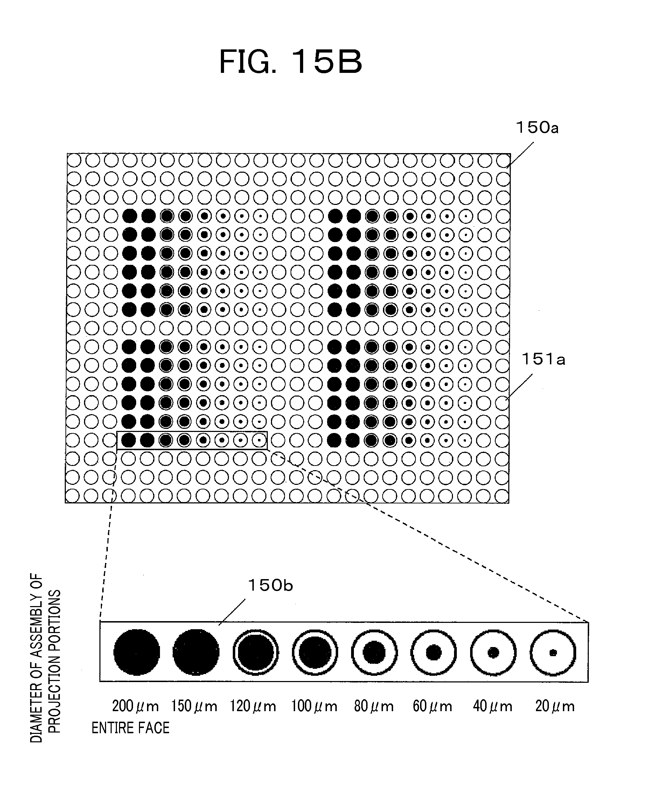

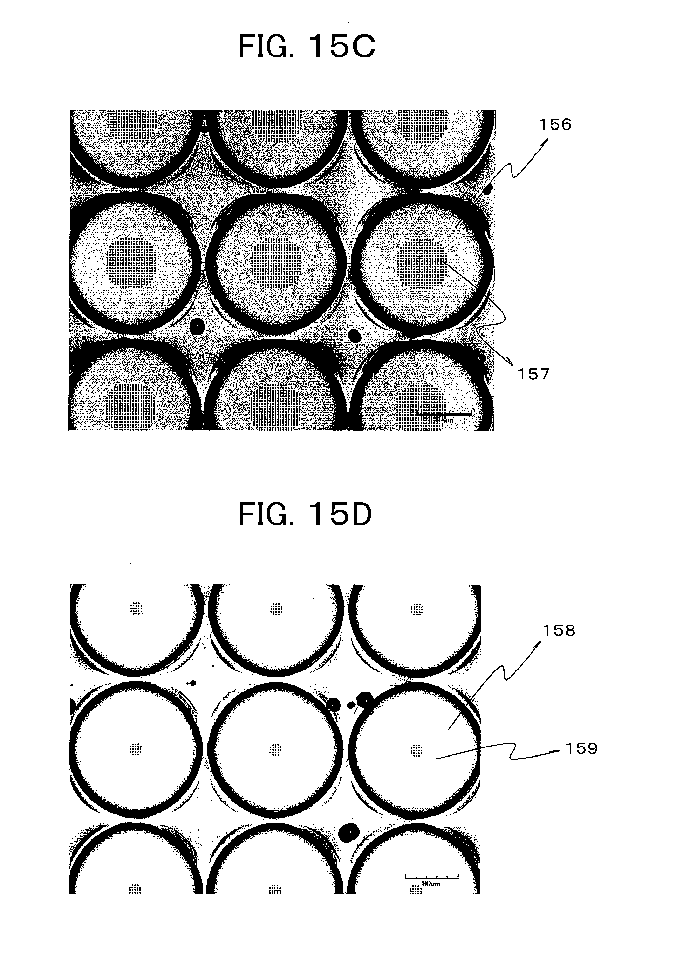

Fig. 15A, Fig. 15B, Fig. 15C, and Fig. 15D show the culture sheet of Example 5. In Example 5, among the various variants shown in Example 4 of Fig. 8, an example is shown in which the first arrangement pattern 125a is a flat structure, and a culture sheet having a pattern in which projections are arranged in the central portion of the hole is applied to the chamber slide which is a culture sheet retaining member. That is, in this Example, by providing a culture sheet having a structure in which the culture regions consist of the first region and the second region surrounding the same, projections are arranged on in the first region, and projections are not formed in the second region, spheroids which are 3D tissues having similar diameters are retained in the central portion of the culture region corresponding to the first region, whereby the spheroids can be retained in the target position.

Herein, an example in which projections are arranged near the center in the culture region is shown, but the center need not be necessarily included, and it goes without saying that projections may be arranged in a desired region in the culture region. Moreover, although an example in which the projection region of an approximate rhombus shape and circle shape is formed is shown, it goes without saying that the projection region may be in the shape of a square or a polygon.

Fig. 15A shows an example of the culture sheet prepared in this Example. Fig. 15A shows one structure of holes 151 constituted by a plurality of partition structures 152 in a single culture sheet. The configuration that the inside of the hole 101 constitutes a culture region by a cell tissue formation unit partitioned by a partition wall is the same as in the above-mentioned Example. A plurality of projections 153 retained at the bottom 154 of the hole 151 includes a plurality of microprojections. Moreover, the diameter of this hole 151 is set tube a hole diameter 155. Preferably, in the culture sheet 150, the hole 151 including the above-mentioned partition wall 152 and a plurality of projections 153 formed within the hole 151 are formed from the same material integrally. It should be noted that the shape of this hole 151 is not limited to round, but may be another shape such as a square, as in the above-mentioned Example.

In a suitable aspect of this Example, as shown in the culture sheet 150a of Fig. 15B, culture sheets having a pillar height, pillar diameter, and pillar pitch of 1.0 µm, 1.0 µm, 2.0 µm and, 1.0 µm, 2.0 µm, 4.0 µm, respectively, and a diameter of the assembly of projection portions of 200 µm (nanopillars on the entire surface), 150 µm, 120 µm, 100 µm, 80 µm, 60 µm, 40 µm, 20 µm can be used.

As shown in an enlarged portion 150b of Fig. 15B, providing a culture substrate in which the formation region (constitutional proportion) of projections by in the hole, that is, a cell tissue formation unit partitioned by a partition wall constituted in multi-stages is effective as a test substrate for grasping an optimum formation rate of projection regions in Example 7 described later. An optimum constitutional proportion may vary depending on the cell strains and desired size intended for culture, and therefore performing a culture test in advance using such a culture substrate is useful since it affects the culture efficiency thereafter. It should be noted that the hole 151a indicates a hole in which no projection is formed.

The culture sheet 150a is formed from a material which does not adversely affect cells, and it is, in this example, polystyrene. However, it goes without saying that the material is not limited to polystyrene.

As a typical example, a SEM image in which the diameter of the assembly of projection portions is 80 µm is shown in Fig. 15C, and an SEM image in which the diameter is 20 µm is shown in Fig. 15D. In each of Fig. 15C, and Fig. 15D, 156 and 158 represent a hole, while 157 and 159 represent a projection assembly.

In this manner, the hole 151 including the partition wall 152 and the plurality of projections 153 formed inside the hole 151 are formed integrally from a single material which has no adverse effects on cells as culture sheets 150, 150a, whereby cells can be grown with no foreign substances adhering to cells in the culture step.
Furthermore, since cells are grown in each of the partitions, cells of a homogeneous size can be formed.

Moreover, a plurality of projections are provided within the partition wall 152 arranged in a surrounding manner. Therefore, cell movement, which is the ability inherent to the cells, is promoted, and cells are grown by the movement so that a cell culture which can maintain the cell activity is possible with no influence of disturbance (stress) by rotation culture or the like.

When a culture region is to be formed while these holes 151 and projection assembly 153 are provided separately, they need to be joined by adhesion or welding. For example, when these are joined by adhesion, adhesive components enter into the culture region, which may adversely affect generated cells.
In joining by welding, the inner diameter of the hole 151 is a hyperfine region diameter on the cell formation level, and therefore it is very difficult to perform welding while forming a target cell region and not damaging the partitions and projections. When the partitions and projections have damages and deformations, unwanted stress may be applied on the cells in the process of cell formation, and the movement of the cells themselves may be impaired.

Therefore, also in this Example, as stated above, the hole bottom 154, partition wall 152 and projections 153 constituting the holes 151 which form the culture region are preferably formed integrally by forming integrally in such a manner, it is preferable because culture excluding the influence of unwanted components other than those required for cell culture can be performed.

In this Example, a culture sheet in which the pillar diameter, pillar pitch and pillar height are 1.0 µm or 2.0 µm, 2.0 µm or 4.0 µm, 1.0 µm, respectively, was used, but as will be described later, the culture sheet may be one with other specifications than these. The height of the partition structure is 70 µm in this Example, but this value is not necessarily used, and suitably the height may be such that the formed cells do not get over the partition.

The culture sheets 150, 150a in this Example are produced by a method similar to that in Example 1, and therefore detailed description of the production method will be omitted herein. In addition, also in this Example, the chamber slide 109 with the culture sheet 150 affixed as shown in Fig. 3 can be produced, and it goes without saying that a chamber slide having an overall constitution and a principal part cross section similar to those in Fig. 13A, Fig. 13B, Fig. 13C can be obtained, and therefore explanation will be omitted herein.

### Example 6

Subsequently, Example 6 will be described with reference to Figs. 4 and 5. This Example shows the constitution of a multiwell plate with a culture sheet using the culture sheets 150, 150a described in Example 5, and a production example thereof. The constitution of the multiwell plate and a production example of the same have been described in Figs. 4 and 5, but this Example is basically similar to Example 2 except that the culture sheets 150, 150a are used in place of the culture sheet 100 used in Example 2.

Fig. 4(a) is a bottom view of the frame body 111 constituting the multiwell plate. The frame body 111 which is a culture sheet retaining member is such that has 24 cylindrical hole portions 111a in total, arranged in 4 rows and 6 columns, are formed in an area measuring about 125 mm in width, about 80 mm in length, and about 20 mm in height. The material used is polystyrene.

The number of holes formed on the frame body normally ranges from 6 to 1536, varied depending on the use, and therefore the number of holes on this frame body is not limited to 24. The material of the frame body is not limited to polystyrene either.

In producing the culture substrate, the frame body 111 and the culture sheets 150, 150a in Figs. 15A and 15B are joined by ultrasonic welding. The process and constitution mentioned above are the same as those in Example 2, and their explanation will be therefore omitted herein.

In the culture substrate prepared in this manner, a plurality of holes 151 are formed on the culture sheets 150, 150a used in place of the culture sheet 100 formed at the bottom of the frame body 111, and a plurality of projections constituted at the bottom 154 of the hole are constituted by a plurality of microprojections 153. In the culture sheets 150, 150a, the holes 151 including the above-mentioned partition wall 152 and the plurality of projections 153 formed within the holes 151 are formed from the same material integrally. It should be noted that the shape of this hole 151 is not limited to round, and may have other shapes such as a square shape.

In this manner, the hole 151 including the partition wall 152 and the plurality of projections 153 formed inside the hole 151 are formed integrally from a single material which has no adverse effects on cells as a culture sheet, whereby cells can be grown with no foreign substances adhering to cells in the culture step.
Furthermore, since cells are grown in each of the partitions, cells of a homogeneous size can be formed.
Moreover, a plurality of projections are provided within the partition arranged in a surrounding manner. Therefore, cell movement, which is the ability inherent to the cells, is promoted, and cells are grown by the movement so that a cell culture which can maintain the cell activity is possible with no influence of disturbance (stress) by rotation culture or the like.

As stated above, also in this Example, the holes 151 which form the culture region and the projections 153 are preferably formed integrally. By forming integrally in such a manner, culture excluding the influence of unwanted components other than those required for cell culture can be favorably performed.

The overall constitution diagram and principal part cross-sectional view of the multiwell plate with the culture sheet of this Example are also as shown in Figs. 14A, 14B, 14C, 14D as in Example 2, and explanation will be therefore omitted herein.

### Example 7

Subsequently, Example 7 shows an example of application of cells to tissue culture using the culture substrate produced in Examples 5 and 6. An example of application of cells to tissue culture using the culture substrates produced in Examples 1 and 2 was shown previously as Example 3 using Figs. 6 and 7. The difference between this Example and Example 3 is that a culture substrate in which the culture sheets 150, 150a are used in place of the culture sheet 100 is used. Since explanation is common for other point, explanation will be therefore omitted herein.

It should be noted that in this Example, the inoculation density of hepatocytes is set to 5 × 10⁵ cells/ml, but is not limited to this concentration. Herein, the culture sheets 150, 150a used for culture as previously explained, have a pillar height, pillar diameter and pillar pitch of 1.0 µm, 1.0 µm, 2.0 µm and, 1.0 µm, 2.0 µm, 4.0 µm, respectively, but the values are not limited to these.

Moreover, the concentration of Type I collagen added to the culture sheets 150, 150a was set to Example 100 (ng/ml) in this Example, but may be a concentration other than this. Spheroids may be formed at a concentration other than this concentration depending on the conditions of the cells. In this Example, as shown in Fig. 15B, culture sheets having a pillar height, pillar diameter and pillar pitch of 1.0 µm, 1.0 µm, 2.0 µm and, 1.0 µm, 2.0 µm, 4.0 µm, respectively, a hole diameter of 200 µm, a diameter of the assembly of projection portions of 200 µm (nanopillars on the entire surface), 150 µm, 120 µm, 100 µm, 80 µm, 60 µm, 40 µm, 20 µm, respectively was used.
Needless to say, the hole diameter and the diameter of the assembly of projection portions are not limited to these values.

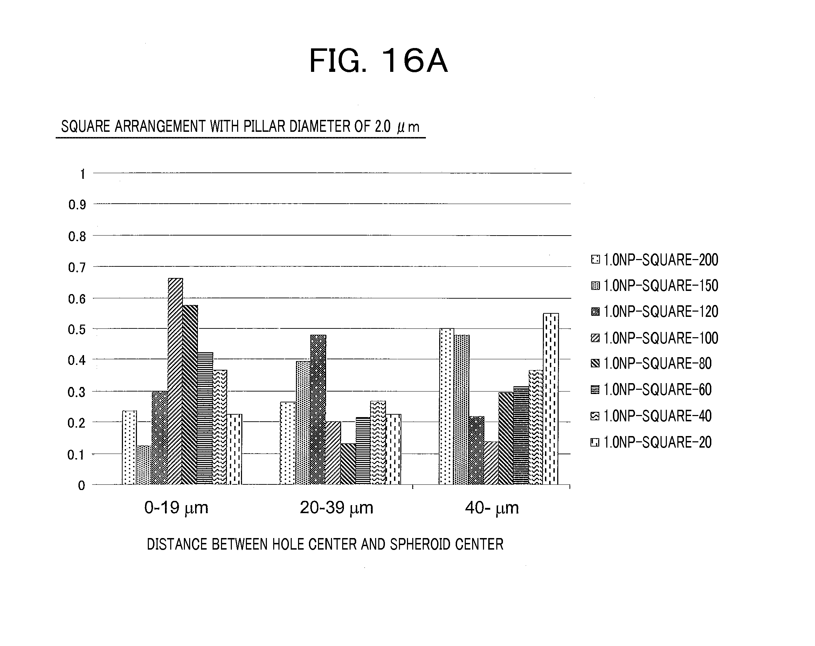

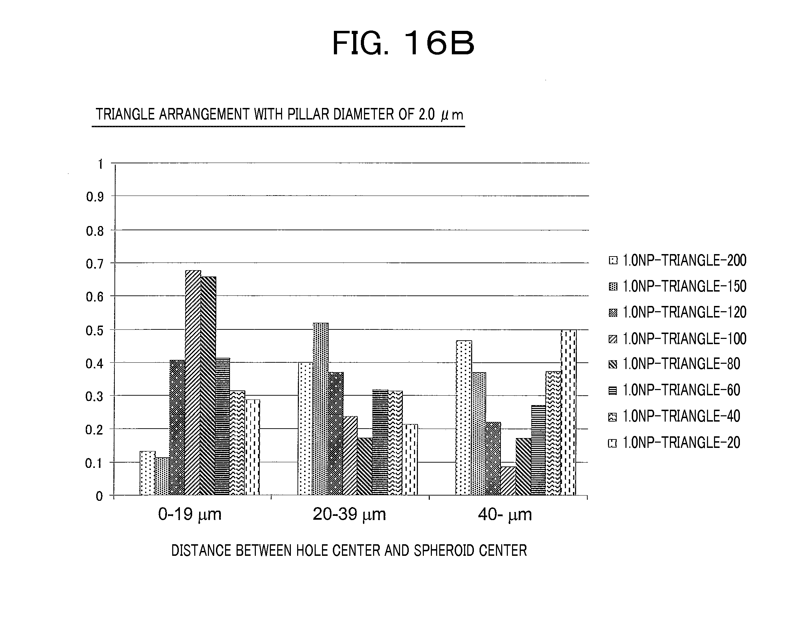

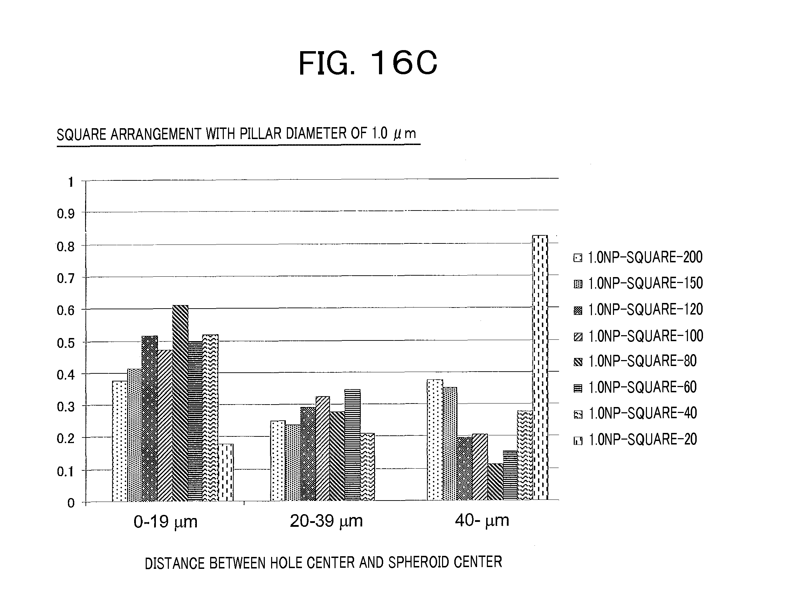

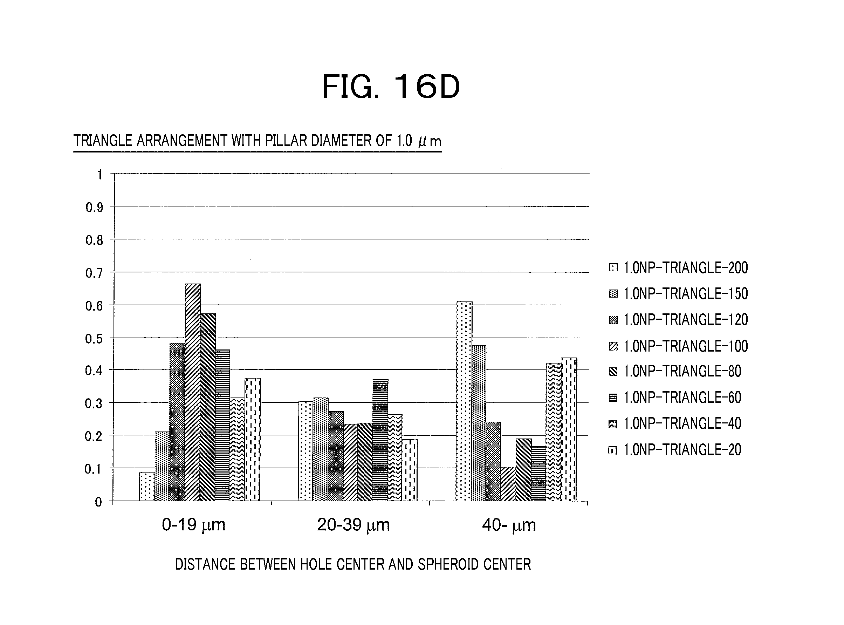

Figs. 16A, 16B, 16C, 16D show the results of the cell culture for 96 hours in total using the culture sheets having these patterns. That is, the graphs of the results of measuring the distance between the center of the hole and the center of the spheroid and verifying the hole center retention rate of the spheroids are shown. Figs. 16A, 16B, 16C, 16D, as illustrated, correspond to a square arrangement with a pillar diameter of 2.0 µm, a triangle arrangement with a pillar diameter of 2.0 µm, a square arrangement with a pillar diameter of 1.0 µm, and a triangle arrangement with a pillar diameter of 1.0 µm, respectively.

The distance between the centers of the hole center and spheroid is indicated in 3 steps of 0 to 19 µm, 20 to 39 µm, 40 µm or more on the horizontal axis of each graph, and the proportion of the number of spheroids occupying each range in the total number of spheroids is indicated on the vertical axis. As a result, in all of the patterns examined at this time, the sheets having a diameter of the assembly of projection portions of 100 µm or 80 µm had higher rates that spheroids are retained closer to the center.

It has been shown that an optimum rate of the diameter of the assembly of projection portions relative to the hole diameter is 40% to 50%, but it is not limited to this value depending on the cell strain and culture conditions. According to the experiment results, when the rate is from 20% to 75%, more than half of the spheroids also fell within the range of the distance between the hole center and the center of spheroid from 20 to 39 µm. The rate may be therefore within this range from 20% to 75%.

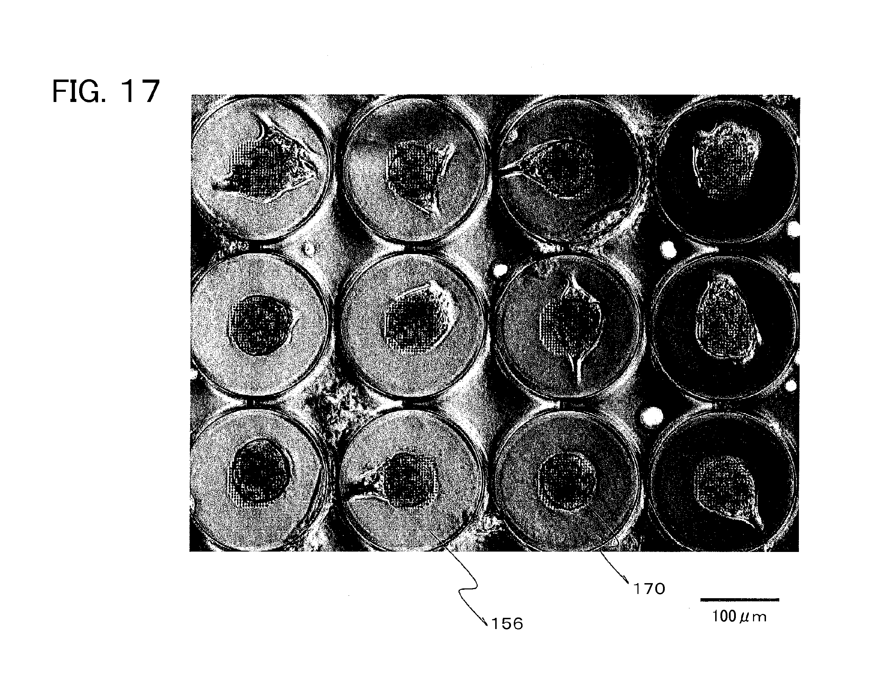

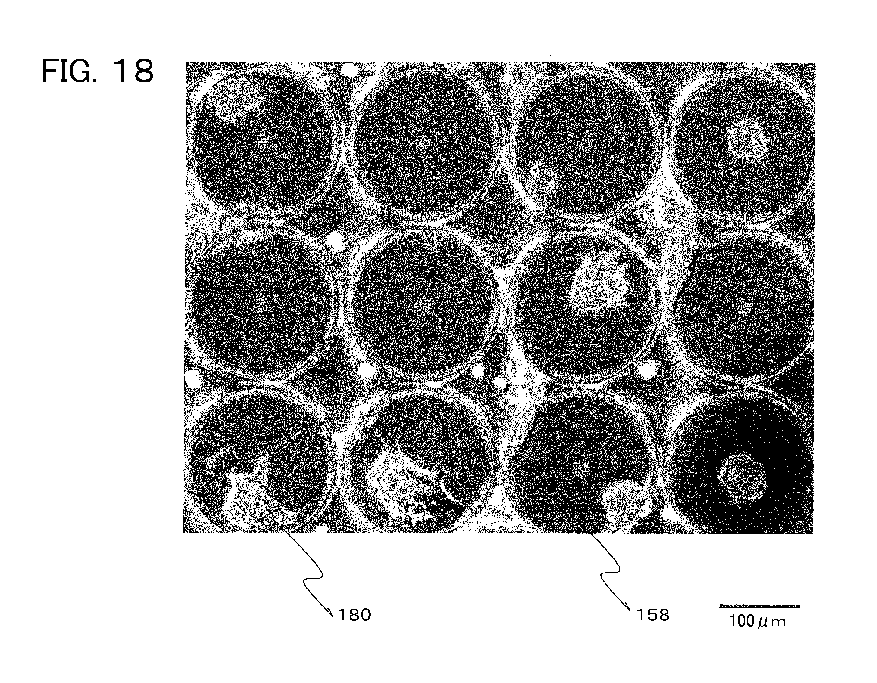

Figs. 17 and 18 show the results of the culture sheets in which a pillar height, pillar diameter, and a pillar pitch of 1.0 µm, 2.0 µm, 4.0 µm, respectively, and a diameter of the assembly of projection portions of 80 µm and 20 µm, respectively, as typical examples of phase-contrast micrographs of the results of culture of the culture sheet of this Example. The numbers 170, 180 in the holes 156, 158 in the figures represent spheroids. As shown in Fig. 17, when the diameter of the assembly of projection portions is 80 µm, the spheroids 170 having almost the same diameter were retained in the central portions of the holes 156. In contrast, as shown in Fig. 18, in the sheet having a diameter of the assembly of projection portions of 20 µm the spheroids 180 were not retained in the central portions.

As can be clearly seen from the results described above, it was found that according to the culture substrate and culture sheet of the present invention, spherical 3D tissues having such similar sizes are formed without applying any special chemical on the surface of the culture sheet and by stationary culture which causes little stress on cells, and spheroids having similar sizes are retained in the central portions of the holes by appropriately setting the hole diameter and the diameter of the assembly of projection portions.

### Reference Signs List

100, 123, 126, 128, 130, 131, 132, 133, 150, 150a...Culture sheet
101, 124, 151, 156, 158...Hole
102, 152...Partition wall
103, 153, 157, 159...Projection / projection assembly
104, 154...Hole bottom
105, 155...Hole diameter
106...Pillar diameter
107...Pillar pitch
108...pillar height
109...Chamber slide
110...Surgical glue
111...Frame body
111a...Hole portion formed on frame body
112...Projection for fixing film
113...Rib structure
114...Culture sheet hole
115...Cell culture plate
116...Type I collagen solution
117...Decompression container
118...Decompression pump
119...Saline for washing (PBS(-))
120...medium
121...Hepatocyte
122...Hepatocyte spheroid
125a, 125b, 127a, 127b, 129a, 129b, 129c...Projection arrangement pattern
1111...Non-slip portion
1112...Joint
1113...Cut face

## Claims

1. A culture substrate for culturing cells, the culture substrate comprising a culture sheet, and a culture sheet retaining member which retains the culture sheet,
the culture sheet having a culture region, the culture region having a plurality of projections formed therein, a partition which partitions the culture region and is taller than the projections being formed, and the constitutional proportion of the projections in the culture region being in the range from 20% to 75%.

2. The culture substrate according to claim 1, wherein the culture substrate has at least one frame surrounding the culture sheet.

3. The culture substrate according to claim 2, wherein the frame is in contact with the culture sheet retaining member.

4. The culture substrate according to claim 1, wherein the sheet retaining member has at least one hole portion, and the culture sheet is constituted at the bottom of the hole portion.

5. The culture substrate according to claim 4, wherein the sheet retaining member has a protrusion formed at the bottom thereof, and the protrusion and the culture sheet are welded.

6. The culture substrate according to claim 2, wherein the frame body has a square or round shape.

7. The culture substrate according to claim 4, wherein the hole portion has a square or round shape.

8. The culture substrate according to claim 1, wherein the culture sheet has a plurality of the culture regions.

9. A culture sheet for culturing cells, the culture sheet comprising a plurality of culture regions,
a plurality of projections formed in each of the culture regions, and
a partition which partitions the culture regions and is taller than the projections, and the constitutional proportion of the projections in the culture region being in the range from 20% to 75%.

10. The culture sheet according to claim 9, wherein, the culture region has a first region and a second region, the width/diameter of the projections in the first region and the width/diameter of the projections in the second region are different.

11. The culture substrate according to claim 1, wherein the partition and a plurality of the projections in the culture sheet are formed integrally from the same material.

12. The culture sheet according to claim 9, wherein, the partition and a plurality of the projections are formed integrally from the same material.

13. The culture substrate according to claim 1, wherein the constitutional proportion of projections in the culture regions is in the range from 40% to 50%.

14. The culture sheet according to claim 9, wherein, the constitutional proportion of projections in the culture regions is in the range from 40% to 50%.

15. The culture sheet according to claim 9, wherein, projections having different constitutional proportions of the projections ranging from 20% to 75% are arranged in each of the culture regions partitioned by the partition.
